## Europäisches Patentamt

(19) **European Patent Office**

**Office européen des brevets**

(11) Publication number: **0 051 399**
**B1**

(12) **EUROPEAN PATENT SPECIFICATION**

(45) Date of publication of patent specification: **07.01.87**

(51) Int. Cl.⁴: **A 61 M 5/34, A 61 M 5/28**

(21) Application number: **81304961.6**

(22) Date of filing: **22.10.81**

(54) **A method of securing an injection needle in a flexible tube.**

(30) Priority: **05.11.80 GB 8035497**

(43) Date of publication of application:
**12.05.82 Bulletin 82/19**

(45) Publication of the grant of the patent:
**07.01.87 Bulletin 87/02**

(84) Designated Contracting States:
**AT BE DE FR IT NL SE**

(56) References cited:
**CH-A- 196 086**
**DE-A-3 143 377**
**DE-C-1 100 883**
**DE-C-1 125 119**
**FR-A- 720 516**
**FR-A-1 575 736**
**FR-A-2 277 652**
**US-A-3 020 631**
**US-A-3 082 498**
**US-A-3 286 314**
**US-A-3 295 176**
**US-A-3 402 436**
**US-A-3 602 954**
**US-E- 25 769**

(73) Proprietor: **COOPERS NEEDLE WORKS LIMITED**
**261/265 Aston Lane**
**Birmingham West Midlands B20 3HS (GB)**

(72) Inventor: **Rowley, Peter William Michael**
**80 Handsworth Wood Road**
**Handsworth Wood Birmingham, B20 2DT (GB)**

(74) Representative: **Stonehouse, Sidney William**
**et al**
**Barker, Brettell & Duncan 138 Hagley Road**
**Edgbaston**
**Birmingham B16 9PW (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

# Description

This invention is concerned with a method of securing an injection needle in a flexible tube, for an injector for medical, lubrication or other purposes, and with an injector manufactured in accordance with the method.

There is a known kind of injector which comprises a pressurised ampoule, in the form of a small glass bottle with an elongate readily-frangible neck, a plastics flexible tube having one end portion sealingly fitted over the bottle neck, and a metal injection needle mounted in, and projecting from, an opposite end portion of the tube. The needle is held in the tube by a plastics plug, which both retains the needle and seals the space between the tube and the needle. In use of this known injector, the neck of the bottle is broken to discharge a liquid (to be injected) from the bottle into the tube and so out through the needle.

That known injector is just one kind of injector in which an injection needle is mounted in a flexible tube, and other kinds of injector, for medical or other purposes, may employ similar arrangements.

The use of a plastics plug to mount the needle in the tube has been found to be disadvantageous in various ways but in particular manufacture and insertion of the plug, to give a reliable seal, can be troublesome. Furthermore, difficulties can arise with the needle picking up plastics material from the plug during assembly of the needle with the plug.

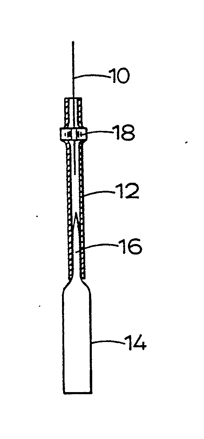

Patent specifications CH—A—196 086 and FR—A—720 516 disclose further examples of needles being secured in flexible tubes by means of some kind of space-filling plug or holder which makes up a difference in diameter between the needle and the tube. In such an arrangement a clamping wire or band can, if necessary, be tightened around the tube to ensure a good seal, as appears to be shown in the single drawing of document FR—A—720 516. Many types of clamping band are known for securing hoses to nipples, for example, a metal band of some kind being simply tightened around the hose to secure and seal the hose on the nipple; one such arrangement is described and illustrated in patent specification US—A—3 082 498, where an outward fold or ear of the band is provided for crimping by means of pincers to draw the band tight (without pinching the hose).

It is the object of this invention to provide an improved method of securing an end portion of an injection needle in a flexible tube, without the use of a space-filling plug or holder to make up the difference in diameter where the needle end portion has an outer diameter significantly smaller than the inner diameter of the tube.

According to the invention a permanently deformable clamping band around the tube is crimped so as to pinch and flatten the tube beside the end portion of the needle, the crimped band retaining the tube so flattened as to secure the needle and seal the tube around the needle.

The tube may conveniently be of a plastics material but could be of any suitable material which will allow the tube to be appropriately deformed in the crimping process and seal against the needle. The band can also be of any suitable material, though some metals may be found to be the most satisfactory materials. The band may or may not be in the form of a continuous loop. Preferably the band is crimped to pinch the tube on each of opposite sides of the needle.

In another of its aspects the invention provides an injector comprising a pressurised ampoule, an injection needle and a flexible tube interconnecting the ampoule and the needle and sealingly fitted to each, characterised in that the needle is secured to the tube by a method as set out in the last preceding paragraph but one.

An injector and its manufacture will now be described, with reference to the accompanying drawing of the injector, to illustrate the invention by way of example.

The injector comprises a steel injection needle 10 inserted in, and projecting from, one end portion of a plastics flexible tube 12, and a pressurised ampoule, in the form of a sealed glass bottle 14, containing a liquid to be injected. As can be seen from the drawing, the needle 10 has an outer diameter significantly smaller than the inner diameter of the tube 12. The bottle comprises an elongate readily-frangible neck portion 16 over which an opposite end portion of the tube is sealingly fitted; in use of the injector the neck portion 16 can be broken to discharge the liquid from the bottle into the tube 12 and so out through the needle 10.

To retain the needle securely in the tube, and to seal between the tube and the needle, a metal clamping band 18 is, in manufacture of the injector, placed around the tube and crimped to pinch and flatten the tube beside the needle.

Other embodiments of the invention could be for use in injecting lubricants, for example for use by a watchmaker, or other liquids.

## Claims

1. A method of securing an end portion of an injection needle (10) in a flexible tube (12), without the use of a space-filling plug or holder to make up the difference in diameter where the needle end portion has an outer diameter significantly smaller than the inner diameter of the tube, in which a permanently deformable clamping band (18) around the tube is crimped so as to pinch and flatten the tube beside the end portion of the needle, the crimped band retaining the tube so flattened as to secure the needle and seal the tube around the needle.

2. A method according to claim 1 wherein the band (18) is crimped to pinch the tube (12) on each of opposite sides of the needle (10).

3. A method according to either of claims 1 and 2 wherein the band (18) is of metal.

4. An injector comprising a pressurised ampoule (14), an injection needle (10) and a flexible tube (12) interconnecting the ampoule and the needle and sealingly fitted to each, characterised in that the needle is secured to the tube by a method according to claim 1.

## Revendications

1. Procédé pour fixer une partie terminale d'une aiguille (10) d'injection dans un tube (12) souple, sans utiliser de bouchon ou d'organe porteur remplisseur de volume (cale d'épaisseur ou adaptateur) pour compenser la différence de diamètre dans la partie terminale de l'aiguille qui possède un diamètre externe nettement plus petit que le diamètre interne du tube, procédé dans lequel une bande (18) de serrage, capable d'une déformation permanente, est plissée ou sertie autour du tube de manière à pincer et à aplatir le tube à côté de la partie terminale de l'aiguille, la bande sertie retenant le tube ainsi aplati de façon à fixer l'aiguille et à rendre étanche le tube autour de l'aiguille.

2. Procédé selon la revendication 1, dans lequel la bande (18) est sertie de façon à pincer le tube (12) sur chacun des côtés opposés de l'aiguille (10).

3. Procédé selon l'une des revendications 1 et 2, dans lequel la bande (18) est en métal.

4. Injecteur comprenant une ampoule (14) contenant un produit sous pression, une aiguille (10) d'injection et un tube (12) souple reliant l'ampoule et l'aiguille et étant fixé de façon étanche à l'ampoule et à l'aiguille, injecteur caractérisé en ce que l'aiguille est fixée au tube par un procédé selon la revendication 1.

## Patentansprüche

1. Verfahren zum Festlegen eines Endabschnitts einer Injektionsnadel (10) in einem Flexiblen Rohr (12), ohne Verwendung eines raumfüllenden Stopfens oder Halters zum Ausgleich der Druchmesserdifferenz dort, wo der Nadel-Endabschnitt einen Außendurchmesser besitzt, der deutlich kleiner ist als der Innendurchmesser des Rohrs, bei dem ein dauernd verformbares Klemmband (18) um das Rohr derart gequetscht wird, daß es das Rohr neben dem Endabschnitt der Nadel einschnürt und abflacht, wobei das gequetschte Band das Rohr derart abgeflacht hält, daß dieses die Nadel festlegt und das Rohr um die Nadel herum abdichtet.

2. Verfahren nach Anspruch 1, bei dem das Band (18) gequetscht wird, um das Rohr (12) auf jeder voneinander abgewandten Seite der Nadel (10) einzuschnüren.

3. Verfahren nach Anspruch 1 oder 2, bei dem das Band (18) aus Metall besteht.

4. Injektor, mit einer unter Druck stehenden Ampulle (14), einer Injektionsnadel (10) und einem flexiblen Rohr (12), das die Ampulle und die Nadel verbindet und dichtend an ihnen anliegt, dadurch gekennzeichnet, daß die Nadel mit Hilfe eines Verfahrens nach Anspruch 1 an dem Rohr festgelegt ist.